# EUROPEAN PATENT APPLICATION

(11) **EP 4 458 481 A1**
(43) Date of publication of application: **06.11.2024**
(21) Application number: 22913653.6
(22) Date of filing: 13.10.2022
(51) Int. Cl.: B07C 5/02, B07C 5/34, B07C 5/36

(54) **MINERAL SORTING SYSTEM**

(30) Priority: 27.12.2021 CN 202111613455
(71) Applicant: Nuctech Company Limited, TongFang Building Shuangqinglu Haidian District Beijing 100084 (CN)
(72) Inventor: LI, Yuanjing, Beijing 100084 (CN); SUN, Shangmin, Beijing 100084 (CN); LIU, Bicheng, Beijing 100084 (CN); LI, Ying, Beijing 100084 (CN); LIU, Lei, Beijing 100084 (CN); ZONG, Chunguang, Beijing 100084 (CN); WANG, Weizhen, Beijing 100084 (CN); HE, Yuan, Beijing 100084 (CN)
(74) Representative: Gunzelmann, Rainer
(86) International application number: PCT/CN2022/125074
(87) International publication number: WO 2023/124389

(57) **Abstract**

A mineral separation system, including a conveyor belt (21) and at least two imaging devices (22). Each imaging device (22) includes a ray source (221) located above the conveyor belt (21) and a ray detector (222) located below the conveyor belt (21). The ray source (221) and the ray detector (222) of each imaging device (22) are arranged facing each other in a direction perpendicular to the conveyor belt (21). The ray sources (221) are staggered in a length direction of the conveyor belt (21) and a width direction of the conveyor belt (21). Projection regions of ray beams emitted by the ray sources (221) do not overlap with each other. Orthographic projections of the ray beams emitted by the ray sources (221) in the length direction of the conveyor belt (21) have an overlapping portion.

## Description

### TECHNICAL FIELD

The present disclosure generally relates to a field of mineral separation technology, and more specifically to a mineral separation system.

### BACKGROUND

In recent years, with a rapid development of economy, a contradiction between supply and demand of mineral resources has become increasingly prominent, and high-grade, easy-to-select, and easy-to-smelt mineral resources have been increasingly reduced. In order to improve a comprehensive utilization of mineral resources, save resources, and ensure a provision of high-quality mineral products, it has become an urgent need to effectively utilize mineral resources such as associated minerals, fine-grained minerals and low-grade minerals and improve a separation efficiency of such mineral products.

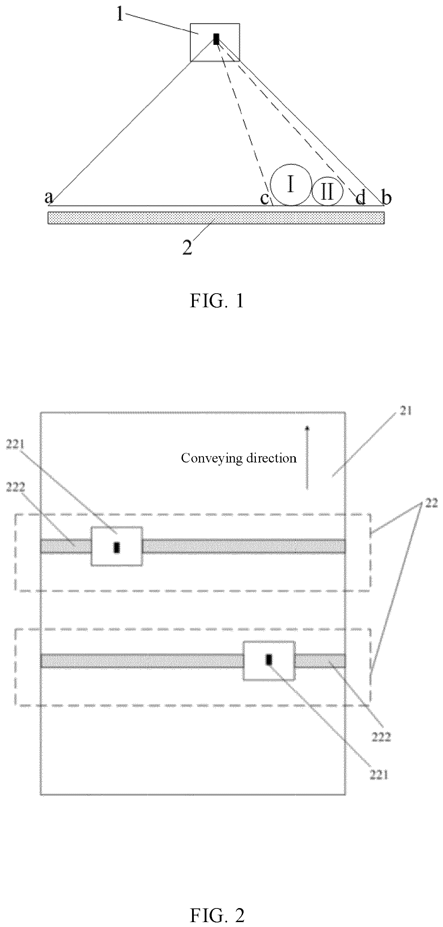

At present, X-ray transmission imaging technology (XRT) is used for separation of mineral products. Referring to FIG. 1, an existing mineral separation system includes a ray source 1, a detector 2, a conveyor belt, a blowing device, and a control device. A principle lies in that: the ray source 1 generates X-rays and irradiates an ore block to be separated on the conveyor belt, transmitted rays are received by the detector 2 for imaging; as different mineral components in the ore block have different attenuation coefficients for the rays, an image of the ore block obtained under the irradiation of the ray source 1 of the same power has a distribution of different optical densities corresponding to different components in the ore block, then the control device analyzes and position the mineral components accordingly, and determines, according to a predetermined threshold, an ore block that needs to be separated, which is then blown and separated by the blowing device.

Referring to FIG. 1, a region ab is an operating region of the conveyor belt. The ore block to be separated is placed in the region ab. If two ore blocks (such as ore block I and ore block II) are arranged adjacent to each other on the conveyor belt, the ore block I and the ore block II may block each other since there is only one ray source 1. When X-rays irradiate the ore block I and the ore block II, a projection region on the detector is a region cd, and the detector 2 may receive a superposition information of the ore block I and the ore block II. It may fail to obtain the information of the ore block I and the information of the ore block II separately, and fail to accurately analyze the two ore blocks separately, which affects an accuracy of a composition analysis. Further, it may fail to accurately determine a position of each ore block in a width direction of the conveyor belt, which affects an accuracy of positioning blowing.

### SUMMARY

In view of the above defects or deficiencies in the related art, it is desired to provide a mineral separation system.

Embodiments of the present disclosure provide a mineral separation system, including a conveyor belt and at least two imaging devices, where each of the at least two imaging devices includes a ray source located above the conveyor belt and a ray detector located below the conveyor belt, the ray source and the ray detector of each imaging device are arranged facing each other in a direction perpendicular to the conveyor belt, the ray sources are staggered in a length direction of the conveyor belt and a width direction of the conveyor belt, projection regions of ray beams emitted by the ray sources do not overlap with each other, and orthographic projections of the ray beams emitted by the ray sources have an overlapping portion in the length direction of the conveyor belt.

Further, the conveyor belt has an operating region for placing a mineral to be separated, and an orthographic projection of the operating region in the length direction of the conveying belt falls within the overlapping portion.

Further, the ray source is arranged obliquely with respect to a conveying plane of the conveyor belt.

Further, the mineral separation system includes two imaging devices; and the ray sources of the two imaging device are respectively located on both sides of a symmetry plane in the length direction of the conveyor belt.

Further, the ray sources of the two imaging devices have a same distance to the symmetry plane.

Further, the ray sources of the imaging devices are arranged at equal intervals in the length direction and/or the width direction of the conveyor belt.

Further, the imaging device further includes a collimator configured to collimate a ray beam emitted by the ray source.

Further, the mineral separation system further includes a frame, and the ray source is rotatably connected to the frame.

Further, the mineral separation system further includes a blowing device and a control device, and the control device is electrically connected to the ray source, the ray detector, the conveyor belt and the blowing device.

Further, the blowing device includes a high-pressure nozzle, and the high-pressure nozzle includes blowing holes arranged in an array.

Technical solutions provided by embodiments of the present disclosure have the following beneficial effects.

The mineral separation system provided by embodiments of the present disclosure includes at least two imaging devices. Orthographic projections of ray beams of the imaging devices have an overlapping portion in the length direction of the conveyor belt. A region of the conveyor belt that faces the overlapping region in the width direction is used as an operating region for conveying an ore block to be separated. Each imaging device may image the ore block placed in the operating region. By imaging the same ore block from different angles using different imaging devices, it is possible to improve an accuracy of composition analysis of the ore block and improve a positioning accuracy of the ore block.

### BRIEF DESCRIPTION OF THE DRAWINGS

Other features, objectives and advantages of the present disclosure will become clearer upon reading the detailed description of non-limiting embodiments with reference to the accompanying drawings. In the accompanying drawings:
FIG. 1 shows a schematic structural diagram of a mineral separation system in the related art;
FIG. 2 shows a schematic structural diagram of a mineral separation system provided by an embodiment of the present disclosure;
FIG. 3 shows a schematic diagram of a projection structure in a length direction of a mineral separation system provided by an embodiment of the present disclosure;
FIG. 4 shows a schematic diagram of a projection structure in a length direction of a mineral separation system provided by another embodiment of the present disclosure;
FIG. 5 shows a schematic diagram of a projection structure in a length direction of a mineral separation system provided by still another embodiment of the present disclosure; and
FIG. 6 shows a schematic structural diagram of a mineral separation system provided by an embodiment of the present disclosure for irradiating and imaging adjacent ore blocks.

### DETAILED DESCRIPTION OF EMBODIMENTS

The present disclosure will be further described in detail below in conjunction with the accompanying drawings and embodiments. It may be understood that the specific embodiments described here are just used to explain the relevant invention, but not to limit the invention. It should also be noted that, for sake of description, only the parts related to the invention are shown in the accompanying drawings.

It should be noted that, as long as there is no conflict, embodiments and features in embodiments of the present disclosure may be combined with each other. The present disclosure will be described in detail below with reference to the accompanying drawings and embodiments.

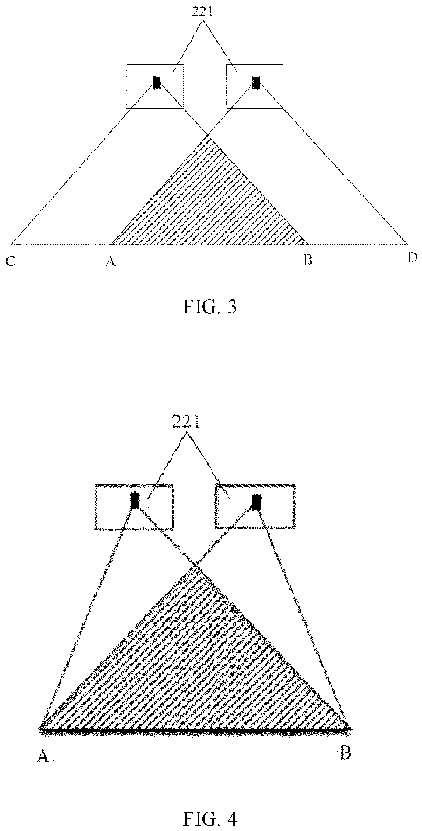

As shown in FIG. 2 and FIG. 3, embodiments of the present disclosure provide a mineral separation system, including a conveyor belt 21 and at least two imaging devices 22. Each imaging device 22 includes a ray source 221 located above the conveyor belt 21 and a ray detector 222 located below the conveyor belt 21. The ray source 221 and the ray detector 222 of each imaging device 22 are arranged facing each other in a direction perpendicular to the conveyor belt 21. The ray sources 221 are arranged in a stagger manner in a length direction and a width direction of the conveyor belt 21. Projection regions of ray beams of the ray sources 221 do not overlap with each other. Orthographic projections of the ray beams emitted by the ray sources 221 have an overlapping portion in the length direction of the conveyor belt 21.

In such embodiments, the mineral separation system includes at least two imaging devices, the orthographic projections of the ray beams emitted by the ray sources of the imaging devices have an overlapping portion in the length direction of the conveyor belt, and the conveyor belt conveys an ore block to be separated in the length direction (i.e., a conveying direction). Thus, an ore block may be imaged at least twice to form at least two images. In the width direction of the conveyor belt, the imaging devices may scan and image the ore block on the conveyor belt from different angles, so that an accuracy of composition analysis and a positioning precision of the ore block may be improved.

Specifically, the conveyor belt has an operating region for placing the ore block to be separated, and an orthographic projection of the operating region in the length direction of the conveying belt falls within the above-mentioned overlapping portion.

For example, the orthographic projection of the operating region in the length direction of the conveyor belt may completely coincide with a bottom of the overlapping portion, or the orthographic projection of the operating region in the length direction of the conveyor belt is a part of the bottom of the above-mentioned overlapping portion.

Referring to FIG. 3, a shaded region illustrates the overlapping portion of the orthographic projections of the ray beams emitted by the two ray sources 221 in the length direction of the conveyor belt 21. The operating region of the conveyor belt 21 for placing the ore block to be separated faces a region AB. Generally, the ore block to be separated in the operating region does not exceed a range of the region AB.

In such embodiments, the ray sources 221 are staggered in the length direction and the width direction of the conveyor belt 21, so that the imaging devices may scan and image the ore block on the conveyor belt 21 from different angles, and the problem of errors in composition analysis and positioning when imaging the ore block using a single ray source from a single angle may be overcome.

Specifically, as the projection regions of the ray beams emitted by the ray sources 221 do not overlap with each other, it may be ensured that the imaging of the imaging devices do not interfere with each other and the imaging regions do not overlap with each other.

In addition, the plurality of imaging devices may scan a same ore block to obtain a plurality of scanning images. A comprehensive analysis may be performed on the plurality of scanning images to obtain an average value of the composition of the ore block, thereby improving the accuracy of composition analysis. The plurality of scanning images may also be used for a comprehensive analysis of positions of ore blocks, which may help to improve a positioning precision of the ore block and an accuracy of positioning blowing, thereby ensuring that the separated ore block meets a separation requirement.

Further, the mineral separation system has a frame (not shown), which may be used to support the conveyor belt 21, the ray source 221 and the ray detector 222.

The mineral separation system further includes a blowing device and a control device. The blowing device is installed on the frame, and the control device is electrically connected to the conveyor belt, the ray source, the ray detector and the blowing device.

Specifically, an orthographic projection of any ray source 221 on a conveying plane of the conveyor belt 21 falls within the conveyor belt 21.

A distance between the orthographic projection of any ray source on the conveying plane of the conveyor belt 21 and a conveying end of the conveyor belt 21 is not less than a predetermined spacing.

At the conveying end of the conveyor belt 21, a blowing device is installed on the frame. The blowing device includes a high-pressure nozzle, and the high-pressure nozzle includes a plurality of blowing holes arranged in an array. The high-pressure nozzle is provided above the conveyor belt 21 and is used to blow a target ore block on the conveyor belt 21. Preferably, the blowing device is movably installed on the frame, so that a blowing position may be adjusted based on a control command of the control device.

In both the width direction and the length direction of the conveyor belt 21, the ray sources are located at inner sides of the conveyor belt 21, so that most of the ray beams emitted by the ray source 221 distributed near an edge of the conveyor belt 21 may irradiate the operating region of the conveyor belt 21. In the width direction of the conveyor belt 21, such design may allow the aforementioned overlapping portion to substantially face a middle region in the width direction of the conveyor belt 21, that is, it may be ensured that the operating region is substantially located in the middle region in the width direction of the conveyor belt 21.

In the length direction of the conveyor belt 21, a distance between the ray source 221 of each imaging device 22 and the conveying end of the conveyor belt 21 is not less than a predetermined spacing. After the imaging device 22 detects an ore block and generates an image for the ore block, the associated control device may analyze the mineral composition according to the image for the ore block, determine a moving speed of the conveyor belt 21 according to an imaging time difference between different imaging devices and the spacing between different imaging devices, and control the corresponding blowing device to blow air before the ore block reaches the conveying end of the conveyor belt 21. If the ray source 221 is located at the conveying end of the conveyor belt 21, it is possible that the composition analysis, the positioning and the separation of the ore block have not been completed when the ore block leaves the conveyor belt 21.

In the aforementioned embodiments, an example in which the blowing device is located at the conveying end of the conveyor belt 21 is shown. However, according to actual needs, it is also possible to provide a blowing device (hereinafter referred to as an intermediate blowing device) between the plurality of imaging devices 22. In this way, it is possible to perform a primary blowing of ore blocks according to a scanning result and requirement of an imaging device located before the intermediate blowing device. After the primary blowing, it is possible to perform a composition analysis and positioning again by using an imaging device located after the intermediate blowing device, and perform a secondary blowing of ore blocks according to a corresponding scanning result and requirement, so that the accuracy of mineral separation may be further improved.

Furthermore, the ray source 221 may be arranged obliquely with respect to the conveying plane of the conveyor belt 21, so that a size of the overlapping portion of the ray beams emitted by the ray sources 221 in the imaging devices 22 may be adjusted.

As an optional embodiment, the mineral separation system includes two imaging devices 22, and the ray sources 221 of the two imaging devices 22 are respectively located on both sides of a symmetry plane in the length direction of the conveyor belt 21. Such design may allow the aforementioned overlapping portion to substantially face the middle region in the width direction of the conveyor belt 21, so as to ensure that the operating region is substantially located in the middle region in the width direction of the conveyor belt 21, prevent the operating region from being biased to one side of the conveyor belt, and ensure that the conveyor belt 21 may convey the ore block smoothly.

Specifically, it is preferable that the ray sources of the two imaging devices 22 have a same distance to the symmetry plane, so that the operating region is located in the middle region in the width direction of the conveyor belt 21.

Referring to FIG. 3, a shaded region represents the overlapping portion of the orthographic projections of the ray beams of the two ray sources 221 in the length direction of the conveyor belt 21. An ore block located in the shaded region of the conveyor belt may be successively detected by the two imaging devices, an ore block located in a region CA of the conveyor belt may only be detected by a left imaging device 221, and an ore block located in a region BD of the conveyor belt may only be detected by a right imaging device 221. Therefore, the region AB is preferably used as the operating region of the conveyor belt for placing the ore block to be separated.

Furthermore, the mineral separation system includes at least two imaging devices 22, and the ray sources 221 of the imaging devices 22 are arranged at equal intervals in the width direction of the conveyor belt 21. Such design may allow the aforementioned overlapping portion to substantially face the middle region in the width direction of the conveyor belt 21, so as to ensure that the operating region is substantially located in the middle region in the width direction of the conveyor belt 21, prevent the operating region from being biased to one side of the conveyor belt, and ensure that the conveyor belt may convey the mineral block smoothly.

Furthermore, the ray sources 221 of the imaging devices 22 are arranged at equal intervals in the length direction of the conveyor belt 21.

FIG. 4 shows a schematic diagram of a projection structure in a length direction of a mineral separation system provided by another embodiment of the present disclosure.

Referring to FIG. 4, the mineral separation system provided by such embodiment differs from the mineral separation system in the embodiment of FIG. 3 in that each imaging device 22 further includes a collimator (not shown). The collimator faces the ray source in a direction perpendicular to the conveying belt 21. The collimator is arranged between the ray source 221 and the conveyor belt 21 to collimate a ray beam emitted by the ray source 221.

As shown in FIG. 4, a right field angle of a right ray source 221 may be reduced using a collimator, and a left field angle of a left ray source 221 may be reduced using a collimator, so that the field angles of the two ray sources 221 may be limited within the region AB covering the conveyor belt.

The imaging device using the collimator is suitable for a conveyor belt with a narrow width, and the region AB as the operating region may cover an entire conveying surface of the conveyor belt.

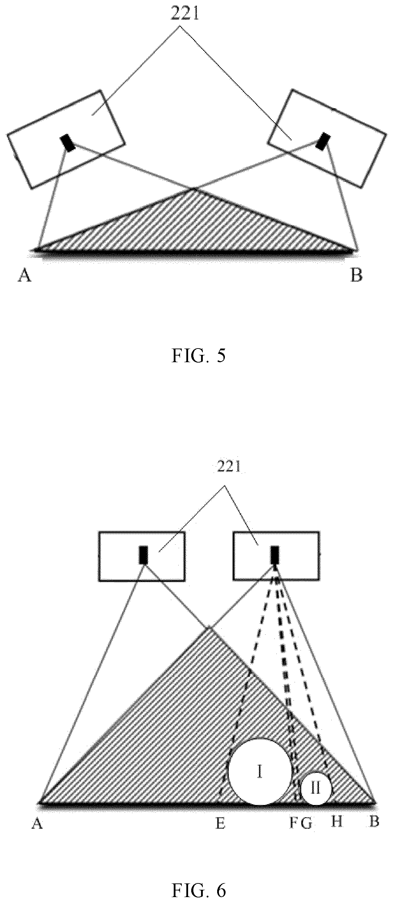

FIG. 5 shows a schematic diagram of a projection structure in a length direction of a mineral separation system according to another embodiment of the present disclosure.

Referring to FIG. 5, the mineral separation system provided by such embodiment differs from the mineral separation system in the embodiment of FIG. 3 in that the ray source is rotatably connected to the frame.

For example, a ray source bracket is provided on the frame, and the ray source is hinged to the ray source bracket. The ray source is locked to the ray source bracket through a locking member (such as screws/bolts).

As shown in FIG. 5, by rotating both ray sources at a certain angle, it is possible to limit the ray beams of the two ray sources within the region AB covering both ends of the conveyor belt.

Similarly, by adjusting the angle of the ray sources, the ray sources are suitable for a conveyor belt with a narrow width, and the region AB as the operating region may cover the entire conveying surface of the conveyor belt.

FIG. 6 shows a schematic structural diagram of a mineral separation system provided by an embodiment of the present disclosure for irradiating and imaging adjacent ore blocks.

The mineral separation system shown in FIG. 4 is used in such embodiments. The mineral separation system includes two imaging devices, each imaging device includes a collimator, and the entire conveying surface of the conveyor belt is the operating region AB.

As shown in FIG. 6, in the operating region AB, for adjacent arranged ore block I and ore block II, when the left ray source 221 is used, the ore block II is partially or completely blocked by the ore block I in the projection direction of the ray beam, so that a ray information received by the corresponding ray detector is a superposition information of the ore block I and the ore block II; when the right ray source 221 is used, the ray information received by the corresponding ray detector includes the information about rays passing through the ore block I and the information about rays passing through the ore block II, so that the ore block I and the ore block II may be distinguished. In this case, a projection region of the ore block I on the ray detector of the right imaging device is EF, and a projection region of the ore block II on the ray detector of the right imaging device is GH. Compared with the use of a single ray source, the use of two imaging devices may analyze the composition of adjacent arranged ore block I and ore block II more accurately, and the accuracy of composition analysis may be improved. Furthermore, based on two imaging devices, it is possible to more accurately obtain a longitudinal vertical projection of the ore block in the length direction of the conveyor belt and a transverse vertical projection perpendicular to the length direction of the conveyor belt, thereby accurately identifying the position of the ore block and improving a positioning blowing precision.

If the mineral separation system shown in FIG. 3 or FIG. 5 is used in such embodiments, it is also possible to clearly distinguish the adjacent arranged ore block I and ore block II. The mineral separation system provided by any of the above-mentioned embodiments may accurately identify the composition of ore block and improve the accuracy of composition analysis; and may accurately identity the position of ore block and improve the positioning blowing precision.

In embodiments of the present disclosure, it is preferred that the ray source is an X-ray emitter. The ray source is used to emit a fan-shaped ray beam, and a field angle of the fan-shaped ray beam may be reduced by a collimator, so that a projection region of the fan-shaped ray beam may be reduced; or the ray source is used to emit a cone ray beam, which may be converted into a fan-shaped ray beam through a collimator.

In the present disclosure, "first", "second", etc. are used to describe various information. However, the information should not be limited to those terms. Those terms are just used to distinguish information of the same type from each other. For example, without departing from the scope of the present disclosure, a first information may also be referred to as a second information, and similarly, a second information may also be referred to as a first information.

In the description of the present disclosure, it should be understood that terms "center", "longitudinal", "transverse", "length", "width", "thickness", "upper", "lower", "before", "after", "left", "right", "vertical", "horizontal", "top", "bottom", "inside", "outside", "clockwise", "counterclockwise", "axial direction", "radial direction", "circumferential direction", etc. are used to indicate orientations or positional relationships shown based on the accompanying drawings, which are intended to facilitate the description of the present disclosure and simplify the description, not to indicate or imply that the device or element referred to must have a specific orientation or must be constructed or operated in a specific orientation, and should not be understood as limitations to the present disclosure.

The above description is just an illustration of preferred embodiments of the present disclosure and technical principles used in the present disclosure. Those skilled in the art should understand that the scope of the invention involved in the present disclosure is not limited to technical solutions formed by a specific combination of the above technical features, but should also cover additional technical solutions formed by any combination of the above technical features or equivalent features without departing from the inventive concept, such as technical solutions formed by exchanging the above features with technical features disclosed in (but not limited to) the present disclosure with similar functions.

## Claims

1. A mineral separation system, comprising:
a conveyor belt; and
at least two imaging devices,
wherein each of the at least two imaging devices comprises a ray source located above the conveyor belt and a ray detector located below the conveyor belt, the ray source and the ray detector of each imaging device are arranged facing each other in a direction perpendicular to the conveyor belt, the ray sources are staggered in a length direction of the conveyor belt and a width direction of the conveyor belt, projection regions of ray beams emitted by the ray sources do not overlap with each other, and orthographic projections of the ray beams emitted by the ray sources have an overlapping portion in the length direction of the conveyor belt.

2. The mineral separation system according to claim 1, wherein the conveyor belt has an operating region for placing a mineral to be separated, and an orthographic projection of the operating region in the length direction of the conveying belt falls within the overlapping portion.

3. The mineral separation system according to claim 2, wherein the ray source is arranged obliquely with respect to a conveying plane of the conveyor belt.

4. The mineral separation system according to any one of claims 1 to 3, wherein the mineral separation system comprises two imaging devices; and
the ray sources of the two imaging device are respectively located on both sides of a symmetry plane in the length direction of the conveyor belt.

5. The mineral separation system according to claim 4, wherein the ray sources of the two imaging devices have a same distance to the symmetry plane.

6. The mineral separation system according to any one of claims 1 to 3, wherein the ray sources of the imaging devices are arranged at equal intervals in the length direction and/or the width direction of the conveyor belt.

7. The mineral separation system according to any one of claims 1 to 3 and 5, wherein the imaging device further comprises a collimator configured to collimate a ray beam emitted by the ray source.

8. The mineral separation system according to any one of claims 1 to 3 and 5, further comprising a frame, wherein the ray source is rotatably connected to the frame.

9. The mineral separation system according to claim 1, further comprising a blowing device and a control device, wherein the control device is electrically connected to the ray source, the ray detector, the conveyor belt and the blowing device.

10. The mineral separation system according to claim 9, wherein the blowing device comprises a high-pressure nozzle, and the high-pressure nozzle comprises blowing holes arranged in an array.
